Europäisches Patentamt

European Patent Office

Office européen des brevets

(11) Numéro de publication: **0 209 416**
**B1**

(12) **FASCICULE DE BREVET EUROPEEN**

(45) Date de publication du fascicule du brevet: **25.04.90**

(51) Int. Cl.⁵: **G 10 K 9/10, G 10 K 11/26**

(21) Numéro de dépôt: **86401248.9**

(22) Date de dépôt: **10.06.86**

(54) Générateur d'impulsions élastiques de grande puissance focalisées dans un liquide et obtenues par percussion.

(30) Priorité: **28.06.85 FR 8509865**

(43) Date de publication de la demande:
**21.01.87 Bulletin 87/04**

(45) Mention de la délivrance du brevet:
**25.04.90 Bulletin 90/17**

(84) Etats contractants désignés:
**BE CH DE GB IT LI NL SE**

(56) Documents cités:
**DE-A-1 548 497**
**DE-A-3 328 039**
**DE-C- 612 569**
**US-A-3 053 220**
**US-A-3 053 338**
**US-A-4 095 667**

(73) Titulaire: **Dory, Jacques**
**91 rue des Molveaux**
**F-77450 Coupvray Esblay (FR)**

(72) Inventeur: **Dory, Jacques**
**91 rue des Molveaux**
**F-77450 Coupvray Esblay (FR)**

(74) Mandataire: **Marquer, Francis et al**
**35, Avenue Victor Hugo**
**F-78960 Voisins le Bretonneux (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

Les ondes élastiques de grande puissance propagées dans un liquide sont utilisées pour détruire des structures solides. C'est le cas en particulier des calculs rénaux ou vésicaux.

Pour que le procédé soit utilisable sur des tissus biologiques, il est nécessaire de pouvoir focaliser l'onde en un point précis, afin de limiter la densité d'énergie en dehors de la zone d'utilisation et de réduire les risques de dommages aux tissus traversés.

Or, on sait que la dimension d'une tache focale est proportionnelle à la longueur d'onde de l'onde élastique, donc à la durée du front d'onde.

Il est donc indispensable, pour obtenir une focalisation efficace, de travailler avec des fronts d'onde très raides, de l'ordre de la microseconde. Dans ce cas, par exemple, l'énergie peut être concentrée sur un diamètre de l'ordre de 3 mm.

Pour résoudre le problème de la génération de fronts d'onde très raides, on a proposé d'utiliser soit des sources ponctuelles d'ondes élastiques, soit des sources réparties.

Les sources ponctuelles sont constituées soit par la mise à feu, dans l'eau, d'une microcharge dont l'explosion engendre une onde sphérique, soit par la décharge d'un arc électrique entre deux électrodes immergées.

Dans les deux cas, l'onde sphérique engendrée est concentrée par un miroir elliptique. Ces solutions procurent un mauvais contrôle de la puissance et de la forme de l'onde produite et entraînent une usure rapide des pièces qu'elles mettent en oeuvre. La fiabilité est faible, du fait que l'intensité très élevée au niveau d'une source ponctuelle entraîne l'apparition de phénomènes perturbateurs.

Les sources réparties ont une grande surface dont tous les points vibrent en phase pour engendrer une onde plane, que l'on concentre, soit avec une lentille acoustique, soit en donnant à la source elle-même la forme d'une calotte sphérique. Elles ont a priori l'avantage d'une fatigue limitée, grâce au fait que la densité d'énergie sur la surface de la source est beaucoup plus faible qu'au point focal.

L'invention porte sur un dispositif qui appartient à cette catégorie.

On connaît déjà des générateurs à source répartie de grande puissance dans lesquels celle-ci est constituée par une mosaïque d'éléments piézoélectriques. Leur inconvénient est que leur surface doit être importante, du fait que la puissance procurée pour chaque élément est relativement faible et que les générateurs électroniques nécessaires à l'excitation des éléments doivent atteindre des puissances instantanées de l'ordre du mégawatt, ce qui conduit à ces coûts élevés.

On connaît par ailleurs, d'après le brevet US-A-3 053 220, un générateur d'ondes élastiques comprenant une pièce soumise à un choc élastique, un projectile, une pièce de propulsion du projectile, un organe moteur de la pièce de propulsion et un organe de rappel de la pièce de propulsion, la pièce soumise à un choc élastique et le projectile ayant deux faces parallèles respectives en regard, la pièce de propulsion entraînée par l'organe moteur communiquant au projectile une énergie cinétique prédéterminée en l'amenant dans un premier temps d'une position de repos à une position active dans laquelle il transmet ladite énergie cinétique à la pièce de propulsion, tandis que l'organe de rappel éloigne ensuite la pièce de propulsion de la pièce soumise à un choc élastique et que, dans un deuxième temps, au cours duquel la pièce de propulsion est arrêtée, le projectile vient en impact avec la pièce soumise à un choc élastique. Ce générateur n'est toutefois pas apte à engendrer des impulsions courtes et à focaliser l'onde dans un liquide.

L'invention se propose de réaliser un générateur d'impulsions courtes de grande puissance à source répartie de réalisation particulièrement simple et permettant la focalisation de l'onde élastique dans un liquide.

Le générateur suivant l'invention comprend une pièce soumise à un choc élastique, un projectile, une pièce de propulsion du projectile, un organe moteur de la pièce de propulsion et un organe de rappel de la pièce de propulsion, la pièce soumise à un choc élastique et le projectile ayant deux faces parallèles respectives en regard, la pièce de propulsion entraînée par l'organe moteur communiquant au projectile une énergie cinétique prédéterminée en l'amenant dans un premier temps d'une position de repos à une position active dans laquelle il transmet ladite énergie cinétique à la pièce de propulsion, tandis que l'organe de rappel éloigne ensuite la pièce de propulsion de la pièce soumise à un choc élastique et que, dans un deuxième temps, au cours duquel la pièce de propulsion est arrêtée, le projectile vient en impact avec la pièce soumise à un choc élastique, et est caractérisé en ce que:

le générateur comprend une enceinte contenant un liquide de transmission des ondes élastiques et ayant une paroi dans laquelle ladite pièce soumise à un choc élastique est rigidement montée, cette dernière ayant une surface émettrice agencée pour transmettre une onde plane dans le liquide,

des moyens de focalisation de cette onde plane dans le liquide sont prévus,

un élément élastique est interposé entre le projectile et la pièce de propulsion, cet élément élastique ayant une impédance acoustique sensiblement plus faible que celle du projectile,

la dimension du projectile parallèlement à la direction de déplacement de la pièce de propulsion est déterminée pour que le temps de propagation aller et retour de l'onde élastique dans le projectile corresponde à la durée de l'impulsion élastique que l'on désire engendrer,

la vitesse de déplacement de la pièce de propulsion est au moins de l'ordre de 10 m/s,

le déplacement du projectile à partir de l'instant où la pièce de propulsion est arrêtée est de l'ordre de quelques centièmes de millimètre.

D'autres particularités, ainsi que les avantages de l'invention, apparaîtront clairement à la lumière de la description ci-après.

Au dessin annexé:

La figure 1 représente schématiquement une première forme d'exécution d'un générateur d'impulsions élastiques à percussion selon l'invention;

Les figures 2 et 3 représentent, respectivement en élévation avec coupe partielle et en plan, un mode de montage de l'enclume sur son support;

Les figures 4 et 5 représentent un circuit de réglage automatique du parallélisme des faces associées du marteau et de l'enclume;

Les figures 6 et 7 représentent les formes d'ondes des signaux en différents points dudit circuit de réglage;

La figure 8 représente un mode d'exécution préféré du marteau et de sa tige d'entraînement;

La figure 9 illustre l'évolution de l'onde de pression dans la plaque impactante du marteau de la figure 8;

Les figures 10 à 12 représentent schématiquement des moyens de visualisation de l'obstacle visé par le générateur et de la tache focale du faisceau d'ondes élastiques engendré; et

Les figures 13 et 14 représentent, respectivement en coupe longitudinale et en coupe transversale, une deuxième forme d'exécution du générateur.

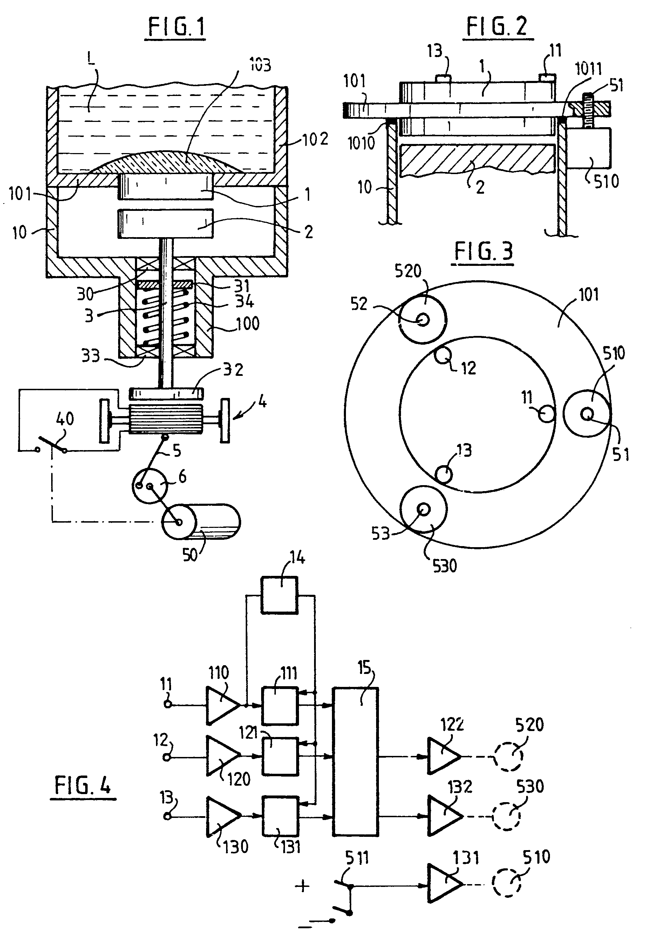

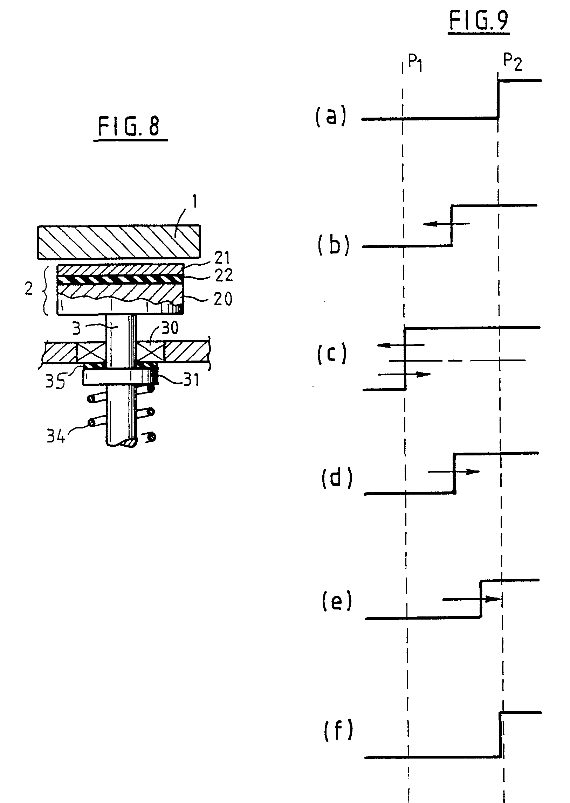

A la figure 1, on a représenté une enclume 1 entourée d'une collerette 101 fixée sur un support cylindrique 10 et coopérant avec un marteau 2 réalisé comme le montre la figure 8, lui-même fixé à une extrémité d'une tige 3 qui coulisse dans deux paliers à billes 30—33 montés dans un manchon 100 qui prolonge le fond du support cylindrique 10. A l'autre extrémité de la tige 3, extérieure au manchon 100, est fixée l'armature 32 d'un électro-aimant 4. La culasse de l'électro-aimant est montée pour pouvoir coulisser suivant l'axe de la tige.

Elle est entrainée en mouvement de translation alternatif par un système bielle-manivelle 5—6, lui-même actionné par un moteur 50. Un interrupteur 40 commande la mise en service de l'électro-aimant. On a symbolisé par une ligne en trait mixte, la commande de cet interrupteur par l'arbre du moteur, par l'intermédiaire d'une came. Un ressort de compression 34 vient s'appuyer sur une butée 31 solidaire de la tige au voisinage du palier 30 et sur le palier 33.

Sur la face de l'enclume 1 opposée à celle qui coopère avec le marteau 2, est accolée une lentille 103 apte à transformer l'onde élastique plane engendrée par la percussion du marteau en une onde sphérique qui se propage dans un liquide L contenu dans une cuve 102 dont le fond est constitué par la collerette 101.

A la mise en service du dispositif, le marteau étant appliqué contre l'enclume par le ressort 34, le moteur 50 amène la culasse de l'électro-aimant en position haute, dans laquelle elle est en contact avec l'armature 32. A ce moment, la came ferme l'interrupteur, si bien que l'électroaimant est excité et que, la culasse effectuant maintenant un mouvement de descente, l'armature est entraînée par elle dans ce mouvement. Le ressort 34 se trouve ainsi comprimé. Au bout d'un parcours réglable, la came ouvre l'interrupteur, si bien que l'électro-aimant libère son armature, et que le ressort entraîne alors le marteau jusqu'à ce qu'il vienne frapper l'enclume.

L'onde sphérique engendrée converge au point focal de la lentille, où s'établit une concentration d'énergie importante.

A titre d'exemple, si le marteau et l'enclume sont en acier, pour une vitesse d'impact de 10 m/s, la pression engendrée sera de $2.10^8$ Pa.

Comme on le précisera ci-après, une couche d'adaptation d'impédances est avantageusement prévue entre l'enclume et le liquide; on peut alors estimer qu'un dixième environ de la pression engendrée sera transmis au liquide. La lentille permet de concentrer la pression au point focal dans un facteur 10 par exemple, ce qui donne une pression de 2 kbar à la tache focale.

Cette pression croît linéairement avec la vitesse d'impact, et sa transmission peut par ailleurs être améliorée en multipliant les couches d'adaptation d'impédance.

Une vitesse d'impact de 10 m/s est facile à réaliser. A titre d'exemple, pour une masse totale en mouvement de 0,1 kg, une distance parcourue de 10 cm et une force de 100 Newtons, la vitesse sera de 14 m/s.

Un facteur de concentration de la pression au point focal de 10 est lui-même obtenu sans difficulté. En effet, pour une impulsion élastique de 1 μs de durée, le diamètre de la tache focale sera de l'ordre 3 mm. Si l'enclume a un diamètre de 5 cm, le facteur de multiplication de la pression est de 16,66.

Il convient toutefois de souligner que les calculs précédents ne sont valables que si tous les points des surfaces qui entrent en contact le font rigoureusement simultanément, faute de quoi l'onde cesse d'être plane et ne peut plus être focalisée, donc la durée de l'impulsion augmente, tandis que son intensité baisse relativement rapidement. Ainsi, pour obtenir un front de montée de 1 μs, avec une vitesse de 10 m/s, la tolérance de planéité et de parallélisme des surfaces coopérantes devra être de l'ordre de ± 5 microns.

Une telle planéité peut être obtenue avec des pièces en acier traité jusqu'au poli optique, mais le parallélisme est par contre difficile à régler, et surtout à maintenir en cours de fonctionnement.

Il est possible, par exemple, en montant le marteau sur rotule et en bloquant celle-ci après application du marteau, d'obtenir un au départ un parallélisme rigoureux à mieux que 1 μ.

Pour garantir le maintien de ce parallélisme malgré les sollicitations importantes subies par le dispositif au cours de son fonctionnement, un dispositif de correction automatique sera de préférence utilisé (il n'est toutefois pas indispensable).

Aux figures 2 et 3, on a représenté l'enclume 1 entourée de la collerette 101. La surface inférieure de cette collerette est fixée par des supports élastiques 1010—1011 (genre silentblocs) de faible compliance sur le bord du support cylindrique 10. La position du plan de la collerette par rapport au plan horizontal peut être ajustée au moyen de trois vis de précision 51-52-53 commandées par des moteurs 510-520-530. Trois capteurs 11-12-13, par exemple du type piézoélectrique, ou jauge de contrainte, sont fixés à la périphérie de l'enclume, sur la face de celle-ci en contact avec la lentille (cette dernière n'a pas été figurée), en regard des vis de réglage respectives. Ces capteurs détectent l'arrivée, sur ladite face de transmission de l'enclume, de l'onde de pression engendrée par le choc sur la face de percussion opposée. Si le marteau et l'enclume ne sont pas rigoureusement parallèles, les fronts avant des impulsions arrivent aux trois capteurs à des instants décalés les uns par rapport aux autres.

A la figure 4, on a représenté un circuit comportant trois amplificateurs 110-120-130 qui reçoivent les signaux des capteurs respectifs. Le front avant de ces signaux déclenche des bascules 111-121-131. Un multivibrateur monostable 14 déclenché par la sortie de l'un des amplificateurs, 110 par exemple, remet simultanément les bascules à zéro, un temps prédéterminé après le choc. On obtient ainsi des créneaux rectangulaires, de largeur variable, qui sont appliqués à un circuit logique 15 agencé pour comparer les largeurs des créneaux issus des bascules 121 et 131 à celle du créneau fourni par la bascule 111, prise comme référence et délivrer des signaux proportionnels aux écarts de largeur. Ces signaux, amplifiés en 122, 132, servent à commander les moteurs 520-530, dans le sens de rotation direct ou rétro- grade suivant le signe des écarts.

La correction de parallélisme s'effectue ainsi progressivement au cours de chacun des chocs successifs et se maintient, au bout d'un certain temps, en permanence. Le moteur 510, commandé, par l'intermédiaire d'un amplificateur 131, à partir d'une source d'excitation qui lui est reliée par l'intermédiaire d'un interrupteur 511, est commandé manuellement, dans le sens direct ou rétrograde, pour faire varier la position moyenne de l'enclume. L'intérêt de ce réglage de position moyenne apparaîtra dans la suite.

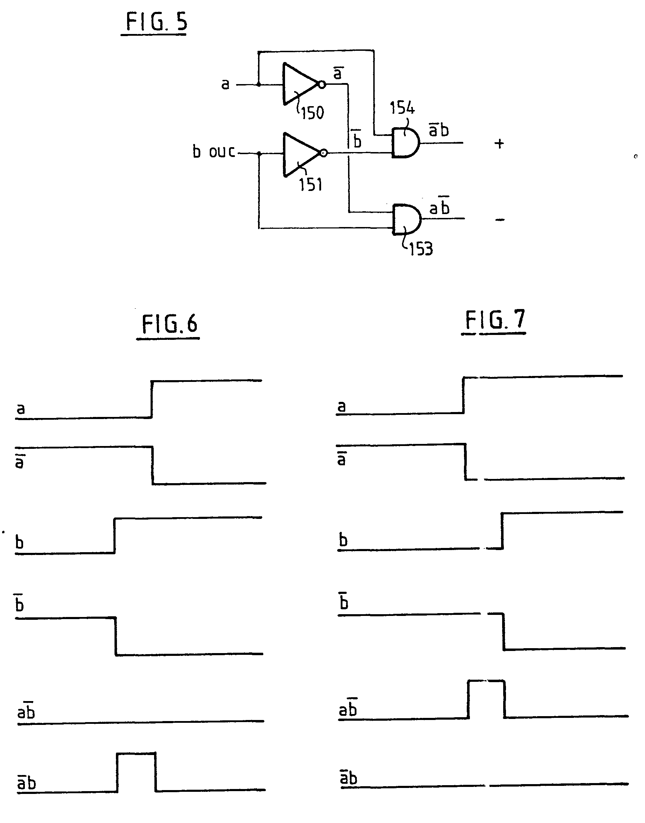

A la figure 5, on a représenté un circuit logique comportant deux inverseurs logiques 150 et 151 qui reçoivent respectivement le signal a issu de la bascule 111 (figure 4) et l'un des deux signaux, par exemple b, issus des bascules 121 et 131. Les signaux de sortie $\bar{a}$ et $\bar{b}$ de ces inverseurs sont appliqués à deux portes ET 153 et 154 qui reçoivent, par ailleurs b, respectivement a et engendrent ainsi les produits logiques respectifs $a\bar{b}$ et $\bar{a}b$.

La figure 6 représente les signaux a, $\bar{a}$, $\bar{b}$, $a\bar{b}$ et $\bar{a}b$ lorsque b est en avance sur a et la figure 7 représente les mêmes signaux lorsque b est en retard sur a.

Dans le premier cas, la sortie $a\bar{b}$ est positive, tandis que la sortie $\bar{a}b$ est nulle, et c'est l'inverse dans le second cas. On peut ainsi commander le moteur 520 dans le sens direct ou rétrograde avec les deux sorties des portes 153 et 154. Un circuit identique, dans lequel l'inverseur 151 recevra le signal c issu de la bascule 131, commandera le moteur 530.

En dehors du premier problème susvisé de maintien du parallélisme entre l'enclume et le marteau, se pose un second problème, à savoir la déformation permanente des pièces qui risque de se produire si la contrainte est appliquée pendant une durée trop importante. Pour réduire le temps de contact entre marteau et enclume à la valeur strictement nécessaire pour engendrer le front d'onde élastique, on utilise avantageusement le dispositif illustré par la figure 8. Le marteau 2 est constitué par une pièce massive 20, de préférence en alliage léger, sur laquelle est fixée une lame d'acier 21 ayant, par exemple, une épaisseur de 3 mm, par l'intermédiaire d'une couche élastique 22. La butée 31, en forme de collerette, vient s'appuyer en fin de course sur le palier 30 par l'intermédiaire d'un joint élastique 35 destiné à éviter le blocage instantané du marteau. Dans cette position de repos, la face externe de la lame 21 du marteau est très proche de l'enclume (par construction du dispositif et par réglage de la position moyenne de l'enclume), à la limite du contact (quelques centièmes de millimètres par exemple). En fin de course de la tige 3, la partie massive 20 du marteau, qui arrive à grande vitesse, est donc bloquée (en 1 ou 2 mm de déplacement) par la coopération des butées 30-31 juste avant de frapper l'enclume. Mais la lame 21, grâce à l'élasticité de la couche 22, continue à progresser par inertie sur un très faible parcours. Une onde de pression se propage alors dans l'enclume 1 et dans la lame 21, et se réfléchit sur la face interne de la lame 21, grâce au fait que l'impédance de ladite lame est beaucoup plus élevée que celle de la couche élastique 22.

Cette onde réfléchie annule la pression incidente, après un temps correspondant à un aller et retour de l'onde élastique dans la lame 21, au bout duquel la pression au niveau de l'enclume se trouve ainsi annulée.

Cet effet, conjugué à la force de rappel de la couche élastique, entraîne une rupture du contact entre le marteau et l'enclume.

Dans la lame 21 de l'exemple ci-dessus, la durée de trajet aller et retour est de l'ordre de 1 $\mu$s, c'est-à-dire égale à la durée théorique de formation du front d'onde. Les contraintes statiques se trouvent pratiquement éliminées.

La figure 9 illustre la propagation de l'onde de pression P à l'intérieur de la lame 21 après l'impact. $P_1$ est la pression sur la face arrière (ou externe), $P_2$ la pression sur la face avant.

En (a), on a illustré le moment de l'impact; en (b) à (f), des temps postérieurs à l'impact, respectivement de 0,25 $\mu$s; 0,5 $\mu$s (instant de réflexion sur la face arrière); 0,75 $\mu$s, 0,9 $\mu$s et 1 $\mu$s.

Un troisième problème de réalisation du générateur d'onde élastique décrit est celui de l'amortissement aérodynamique: juste avant l'impact, une lame d'air se trouve emprisonnée entre le marteau et l'enclume et elle peut provoquer un freinage important. Pour atténuer cet effet, on placera l'ensemble de support du marteau dans une enceinte à vide, ou plus simplement, on ménagera dans l'enclume ou le marteau une multitude de sillons d'évacuation de l'air.

Un quatrième problème est celui de la transmission de l'énergie du liquide. L'impédance de l'acier, où l'onde est engendrée, est environ trente fois supérieure à celle de l'eau, et des lames d'adaptation d'impédance sont nécessaires si l'on veut transmettre une fraction importante de l'énergie. On interposera avantageusement plusieurs lames entre le matériau de l'enclume et l'eau, avec des impédances échelonnées et une épaisseur égale au quart de la longueur d'onde de l'impulsion.

La réalisation de telles lames d'adaptation d'impédance est à la portée de l'homme de l'art.

Pour agir efficacement sur un obstacle localisé, tel que calcul rénal, il est utile de localiser celui-ci avec précision par rapport à la tache focale sphérique et de visualiser la position réelle de cette dernière.

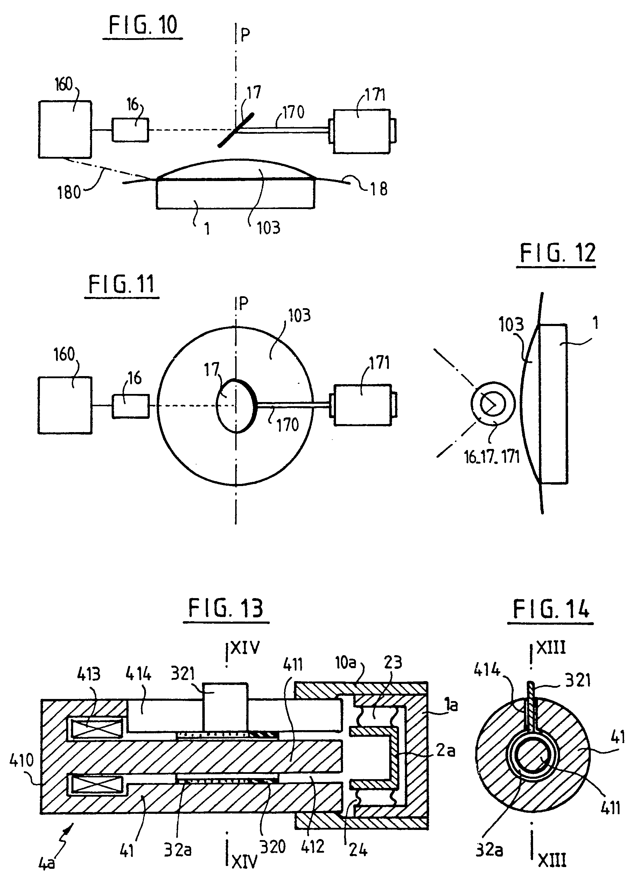

Aux figures 10 à 12, on a symbolisé le générateur d'ondes élastiques par l'enclume 1 accolée à une lentille acoustique 103; une sonde d'échographie 16 coopère avec un miroir 17 orienté à 45° et qui oscille autour d'un arbre 170 de la figure, grâce à un moteur 171.

Le faisceau d'ultrasons engendré par la sonde 16 (associée, bien entendu, à un émetteur d'impulsions électriques approprié qui fait partie d'un dispositif d'échographie symbolisé par le rectangle 160), effectue ainsi un balayage sectoriel dans un plan P perpendiculaire au plan des figures 10 et 11 et passant par l'axe de symétrie du générateur d'ondes élastiques (figure 12). Le dispositif 160 comporte des moyens -connus en soi- de réception et de visualisation des échos formés sur la cible. On obtient ainsi la visualisation de l'obstacle.

Le miroir 17 peut avoir seulement 10 mm de diamètre, par exemple, de façon à n'intercepter qu'une faible partie de l'énergie acoustique émise par le générateur d'ondes élastiques.

Avec un tel dispositif, il est possible de visualiser, en outre, la tache focale du faisceau d'ondes élastiques engendrée. A cet effet, on colle à la surface de l'enclume, une feuille mince d'un polymère piézoélectrique type "PVF$_2$" (18, figure 10) et on la relie (ce qui est symbolisé par la ligne en trait mixte 180) au dispositif échographique 160. On obtient ainsi l'émission d'un faisceau d'ultrasons d'échographie qui aura la même structure géométrique que le faisceau d'ondes élastiques engendré par l'enclume, mais bien entendu, une puissance beaucoup plus faible et une cadence d'impulsion beaucoup plus élevée.

On notera que la feuille de PVF$_2$ a une impédance voisine de celle de l'eau et n'entrave pas la propagation de l'onde de pression produite par l'enclume. Ce matériau, légèrement élastique, est très résistant et peut supporter sans dommage le passage de l'onde de pression.

La feuille de PVF$_2$ permet en outre de contrôler la forme de l'impulsion de pression.

Aux figures 13 et 14, on a représenté une variante d'exécution dans laquelle le marteau 2a est constitué par une coupelle cylindrique creuse de quelques mm d'épaisseur qui coopère avec une enclume de même forme 1a entourée d'un manchon 10a, prolongé au-delà de l'enclume pour venir servir de moyen de support et de centrage de la culasse cylindrique d'un électro-aimant, désigné globalement par le numéro de référence 4a. Cette culasse comprend une pièce cylindrique creuse extérieure 41 fermée à une extrémité par un fond 410 prolongé par un noyau cylindrique axial 411. Ce dernier forme un entrefer 412 avec la pièce 41 et délimite, avec le fond et une portion d'épaisseur plus faible de la pièce 41, un logement annulaire pour une bobine 413 qui reçoit une alimentation permanente.

La pièce 41, le noyau 411 et les coupelles cylindriques 1a et 2d ont leurs axes de symétrie confondus et le bord annulaire du marteau 2a est disposé en regard de l'entrefer annulaire 412, de maniere telle qu'un ensemble cylindrique (monté coulissant autour du noyau 411 dans l'entrefer et constitué par une pièce cylindrique mobile 32a prolongée par un butoir élastique 320) puisse venir faire impact sur le bord du marteau.

Le marteau est attaché à l'enclume au moyen de deux disques métalliques élastiques 23-24 munis d'ondulations circulaires qui autorisent un déplacement de quelques millimètres du marteau suivant l'axe du dispositif, tout en assurant un guidage latéral rigoureux.

La pièce cylindrique 32a est rigide et porte un bobinage alimenté en impulsions par des conducteurs souples, non figurés, tandis que le butoir 320 est légèrement élastique. Il est réalisé, par exemple, en caoutchouc ou silicone. Cet ensemble est relativement léger par rapport au poids du marteau.

La pièce cylindrique creuse 41 est munie d'une fente 414 ouverte suivant deux génératrices extérieure et intérieure, pour loger une lame 321 solidaire de la pièce cylindrique 3 et permettre le coulissement de ladite lame parallèlement auxdites génératrices. Cette lame empêche la rotation de l'ensemble 32a-320 lorsqu'il est propulsé par l'action du champ de l'électro-aimant sur le courant qui parcourt la bobine (impulsion de courant de 1/100 de seconde, par exemple, ayant une polarité convenable pour amener ledit ensemble en position d'impact, et une polarité inverse pour le ramener en position de repos).

A l'instant initial, l'ensemble 32a-320 est en position de repos. Le marteau 2a est alors situé à environ 5 mm de l'enclume 1a par exemple.

Une impulsion appliquée à la bobine 32a projette celle-ci vers la droite.

En fin de course, le butoir 320 vient au contact du marteau et lui transmet l'énergie cinétique de la pièce 32a, en un temps de l'ordre de quelques millisecondes. Le transfert d'énergie doit être total avant que le marteau n'etre en contact avec l'enclume. Le temps de transfert sera déterminé par l'élasticité et la dimension du butoir. Celle-ci est dosée pour éviter le rebond avant transfert.

Le transfert d'énergie entre le marteau et l'enclume ne dure que quelques microsecondes. Comme le transfert d'énergie entre la pièce 32a et le marteau prend un temps environ mille fois supérieur, les pressions engendrées entre ladite pièce et le marteau sont beaucoup plus faibles que celles du choc marteau-enclume.

Il en résulte que la fatigue du dispositif de propulsion est limitée.

On notera que la pression de crête de l'onde élastique engendrée ne dépend que de la vitesse du marteau (et non de sa masse), tandis que la durée de celle-ci est fonction de son épaisseur (temps de parcours aller et retour de l'onde dans le marteau). Avec la solution décrite à la figure 13, la vitesse d'impact peut atteindre 30 m/sec.

L'ensemble du mécanisme décrit est avantageusement logé dans une enceinte vidée d'air, afin de limiter les frottements et surtout l'amortissement aérodynamique du choc final.

Le réglage de la pression engendrée peut se faire en agissant sur l'intensité du courant dans la bobine 32a. La vitesse de déplacement de cette bobine peut être évaluée à chaque instant avec précision -en vue de son contrôle- par mesure de la forme contre-électromotrice induite à ses bornes.

Il doit être bien compris que les deux formes d'exécution décrites et représentées ne sont pas limitatives. Les surfaces coopérantes du marteau et de l'enclume ne sont pas nécessairement égales, ni même planes, et la surface émettrice de l'enclume pourrait avoir une forme appropriée à la focalisation du faisceau.

Par ailleurs, d'autres moyens de désaccoupler la plaquette impactante (21, figure 8 ou 13), laquelle a nécessairement une forme géométrique simple -du dispositif propulseur proprement dit (lequel comprend, à la figure 8, la masse principale 20 du marteau) dès que l'énergie cinétique motrice a été transférée à ladite plaquette impactante- pourront être imaginés. Ils ne comporteront pas nécessairement un élément élastique (tel que 22, figure 8 ou 320, figure 13).

Ce qui importe en définitive, c'est avant tout que l'onde réfléchie ne se propage pas dans l'ensemble propulseur, qu'elle fatiguerait rapidement, et qui a nécessairement des points faibles, sa structure étant relativement complexe et que le transfert d'énergie à la pièce impactante soit beaucoup plus long que le transfert d'énergie de la pièce impactante à l'enclume.

**Revendications**

1. Générateur d'ondes élastiques comprenant une pièce soumise à un choc élastique, un projectile, une pièce de propulsion du projectile, un organe moteur de la pièce de propulsion et un organe de rappel de la pièce de propulsion, la pièce soumise à un choc élastique et le projectile ayant deux faces parallèles respectives en regard, la pièce de propulsion entraînée par l'organe moteur communiquant au projectile une énergie cinétique prédéterminée en l'amenant dans un premier temps d'une position de repos à une position active dans laquelle il transmet ladite énergie cinétique à la pièce de propulsion, tandis que l'organe de rappel éloigne ensuite la pièce de propulsion de la pièce soumise à un choc élastique et que, dans un deuxième temps, au cours duquel la pièce de propulsion est arrêtée, le projectile vient en impact avec la pièce soumise à un choc élastique, caractérisé en ce que:

le générateur comprend une enceinte (102) contenant un liquide (1) de transmission des ondes élastiques et ayant une paroi (101) dans laquelle ladite pièce soumise à un choc élastique (1) est rigidement montée, cette dernière ayant une surface émettrice agencée pour transmettre une onde plane dans le liquide,

des moyens (103) de focalisation de cette onde plane dans le liquide sont prévus,

un élément élastique (22-320) est interposé entre le projectile (21-2a) et la pièce de propulsion (20-32a), cet élément élastique ayant une impédance acoustique sensiblement plus faible que celle du projectile,

la dimension du projectile parallèlement à la direction de déplacement de la pièce de propulsion est déterminée pour que le temps de propagation aller et retour de l'onde élastique dans le projectile corresponde à la durée de l'impulsion élastique que l'on désire engendrer,

la vitesse de déplacement de la pièce de propulsion est au moins de l'ordre de 10 m/s,

le déplacement du projectile à partir de l'instant où la pièce de propulsion est arrêtée est de l'ordre de quelques centièmes de millimètre.

2. Générateur selon la revendication 1, caractérisé par des capteurs (11-12-13) agencés pour détecter les fronts d'onde de pression en des points respectifs distribués à la périphérie de la surface émettrice; des moyens (figure 4) de mesurer les décalages temporels entre les fronts d'onde reçus par les capteurs respectifs et des moyens (510-520-530) d'ajuster, en fonction des décalages temporels ainsi mesurés des vis de réglage (51-52-53) de l'inclinaison de la surface émettrice distribués en des points répartis sur une collerette (101) qui entoure l'enclume, en regard des capteurs respectifs.

3. Générateur selon la revendication 1, caractérisé en ce que la pièce de propulsion est constituée par une pièce inerte (20) sur laquelle est fixée une lame mince (21) qui constitue ledit projectile, par l'intermédiaire d'une couche élastique qui constitue ledit élément élastique et ledit organe moteur comporte une tige (3) solidaire de ladite pièce inerte (20) et comportant un épaulement (31) qui vient s'appuyer en fin de course contre une butée (30) par l'intermédiaire d'un joint élastique (35).

4. Générateur selon la revendication 1, caractérisé par une lentille acoustique (103) accolée à la face émettrice de la pièce soumise à un choc élastique et par au moins une lame d'adaptation d'impédance interposée entre ladite face et ladite lentille.

5. Générateur selon la revendication 4, caractérisé par un dispositif d'échographie (160) associé à une sonde (16) et à des moyens de balayage sectoriel (17-171) permettant la visualisation de la cible visée par l'onde élastique.

6. Générateur selon la revendication 5, caractérisé par une feuille mince d'un polymère piézoélectrique (18) collée à la surface émettrice de la pièce soumise à un choc élastique et reliée au dispositif d'échographie (160).

7. Générateur selon la revendication 1, caractérisé en ce que ladite pièce de propulsion comprend un équipage mobile (32a-320) séparé du projectile et venant en impact avec lui.

8. Générateur selon la revendication 7, caractérisé en ce que ledit équipage mobile est propulsé par des moyens électro-dynamiques (électro-aimant 4a, bobine 32a) et muni d'un butoir (320) relativement élastique.

## Patentansprüche

1. Generator von elastischen Wellen, mit einem einem elastischen Stoss unterworfenen Teil, einem Aufprallelement, einem Vortriebsmittel für besagtes Aufprallelement, einem Organ zur Betätigung des Vortriebsmittels und einem Organ zur Rückstellung des Vortriebsmittels, wobei das einem elastischen Stoss unterworfene Teil und das Aufprallelement zwei parallele, gegenüberliegende Flächen aufweisen und das vom Betätigungsorgan angetriebene Vortriebsmittel dem Aufprallelement eine vorbestimmte kinetische Energie mitteilt, indem es dieses in einer ersten Phase aus einer Ruhestellung in eine aktive Stellung bringt, in welcher es besagte kinetische Energie dem Vortriebsmittel übermittelt und das Rückstellorgan dann das Vortriebsmittel von dem einem elastischen Stoss unterworfenen Teil entfernt und in einer zweiten Phase, während der das Vortriebsmittel angehalten ist, das Aufprallelement auf das einem elastischen Stoss unterworfene Teil auftrifft, dadurch gekennzeichnet, dass:

der Generator ein Behältnis (102) aufweist, mit einer Flüssigkeit (1) zur Übertragung der elastischen Wellen und mit einer Wand (101), in welche besagtes einem elastischen Stoss unterworfenes Teil (1) fest eingebaut ist, wobei besagtes Teil eine Abgabefläche aufweist, zur Übertragung einer ebenen Welle in die Flüssigkeit;

Mittel (103) zur Fokussierung dieser ebenen Welle in der Flüssigkeit vorgesehen sind;

ein elastisches Element (22-320) zwischen dem Aufprallelement (21-2a) und dem Vortriebsmittel (20-32a) angeordnet ist und die akustische Impedanz des besagten elastischen Elementes erheblich geringer ist, als die des Aufprallelementes;

die Abmessungen des Aufprallelementes parallel zur Vorschiebungsrichtung des Vortriebsmittels bestimmt werden, damit die Fortpflanzungszeit der elastischen Welle hin und zurück im Aufprallelement der Dauer des elastischen Impulses entspricht, der erzeugt werden soll;

die Verschiebungsgeschwindigkeit des Vortriebsmittels mindestens bei 10 m/s liegt;

die Fortbewegung des Aufprallelementes vom Augenblick an, wo das Vortriebsmittel angehalten ist, einige hundertstel Millimeter beträgt.

2. Generator nach Anspruch 1, gekennzeichnet durch Erfasser (11, 12, 13), die so angeordnet sind, dass Druckwellenfronten an jeweiligen, am Umfang der Abgabefläche verteilten Punkten erfassen; Mittel (Figur 4), um die Zeitverschiebungen zwischen den von den entsprechenden Erfassern empfangenen Wellenfronten zu messen, und Mittel (510, 520, 530), um, entsprechend den so gemessenen Zeitverschiebungen, Schrauben (51, 52, 53) zur Einstellung der Neigung der Abgabefläche zu justieren, die auf einem Flansch (101) verteilt angeordnet sind, der den Amboss umgibt, und zwar gegenüber den entsprechenden Erfassern.

3. Generator nach Anspruch 1, dadurch gekennzeichnet, dass das Vortriebsmittel von einem massiven Teil (20) gebildet wird, auf dem eine dünne Zunge (21) befestigt ist, welche das Aufprallelement bildet, mittels einer elastischen Schicht, welche das elastische Mittel bildet, und dass besagte Betätigungsmittel eine Achse (3) aufweisen, die fest mit dem massiven Teil (20) gekuppelt ist und eine Schulter (31) aufweist, die am Ende des Hubs mittels einer elastischen Dichtung (35) gegen einen Anschlag (30) anliegt.

4. Generator nach Anspruch 1, gekennzeichnet durch eine akustische Linse (103), die mit der Abgabefläche des einem elastischen Stoss unterworfenen Teils gekuppelt ist,und durch mindestens eine, zwischen besagter Fläche und besagter Linse angeordnete Zunge zur Impedanzanpassung.

5. Generator nach Anspruch 4, gekennzeichnet durch eine Echographievorrichtung (160), die einer Sonde (16) und Mitteln zum abschnittweisen Abtasten (17 - 171) zugeordnet ist, um das von der elastischen Welle angesteuerte Ziel sichtbar zu machen.

6. Generator nach Anspruch 5, gekennzeichnet durch einen dünnen Film aus einen piezoelektrischen Polymer (18), der auf der Abgabefläche des einen elastischen Stoss unterworfenen Teils klebt und mit der Echographievorrichtung (160) verbunden ist.

7. Generator nach Anspruch 1, dadurch gekennzeichnet, dass besagte Vortriebsmittel ein bewegliches System (32a, 320) aufweisen das vom Aufprallelement getrennt ist und auf dieses auftrifft.

8. Generator nach Anspruch 7, dadurch gekennzeichnet, dass besagtes bewegliches System von elektrodynamischen Mitteln (Elektromagnet 4a, Spule 32a) vorgetrieben wird und mit einem relativ elastischen Anschlag (320) versehen ist.

## Claims

1. A generator of elastic waves, comprising a piece subjected to an elastic shock, an impacting element, a means for propelling said impacting element, a means for actuating said propelling means and a means for returning said propelling means, the piece subjected to an elastic shock and the impacting element having two respective parallel faces situated opposite each other, the propelling element, driven by the actuating means, communicating to the impacting element a predetermined kinetic energy by transferring it, in a first stage, from a rest position to an active position in which it transmits said kinetic energy to the impacting element, the return means then separating the propelling means from the piece to be subjected to an elastic shock and, in a second stage, the propelling means being at rest, the impacting element produces an impact on the piece to be subjected to an elastic shock, characterized in that:

the generator comprises an enclosure (102) containing a liquid (L) for transmitting the elastic waves, provided with a wall (101) in which said piece (1) to be subjected to an elastic shock is rigidly mounted, this latter having an emitting surface adapted for transmitting a flat wave in the liquid;

means (103) for focalizing said flat wave in the liquid are provided;

an elastic means (22-320) is interposed between the impacting element (21-2a) and the propelling means (20-32a), said elastic means having an acoustic impedance substantially less than that of the impacting element;

the dimensions of the impacting element parallel to the direction of travel of the propelling means are determined so that the propagation time of the out and home travel of the elastic wave in the impacting element corresponds to the duration of the elastic pulse which it is desired to generate;

the travel speed of the propelling element is at least of the order of 10 m/s;

the travel of the impacting element beginning from the time when the propelling element is stopped is of the order of a few hundredth of millimeter.

2. The generator as claimed in claim 1, characterized by sensors (11, 12, 13) adapted for detecting the pressure wave fronts at respective points distributed at the periphery of the emitting surface; means (Figure 4) for measuring the time shifts between the wave fronts received by the respective sensors and means (510-520-530) for setting, as a function of the time shifts thus measured, screws (51, 52, 53) for adjusting the slant of the emitting surface, distributed at points spaced apart over a collar (101) which surrounds the anvil, opposite the respective sensors.

3. The generator as claimed in claim 1, characterized in that said propelling means is formed by a solid piece (20) on which is fixed a fine plate (21) which forms said impacting element, with interpositioning of a resilient layer which forms said elastic element, and in that said actuating means comprise a rod (3) fast with said solid piece (20) and comprising a shoulder (31) which comes to bear at the end of travel against a stop (30) through a resilient seal (35).

4. The generator as claimed in claim 1, characterized by an acoustic lens (103) coupled to the emitting surface of the piece subjected to an elastic shock and by at least one impedance matching plate interposed between said face and said lens.

5. The generator as claimed in claim 4, characterized by an echograph device (160) associated with a probe (16) and with sectorial scanning means (17-171) for visualizing the target at which the elastic wave is aimed.

6. The generator as claimed in claim 5, characterized by a thin sheet of a piezoelectric polymer (18) bonded to the emitting surface of the piece subjected to an elastic shock and connected to the echograph device (160).

7. The generator as claimed in claim 1, characterized in that said propelling means comprise a mobile assembly (32a-320) separate from the impacting element and impacting same.

8. The generator as claimed in claim 7, characterized in that said mobile assembly is propelled by electrodynamic means (electromagnet 4a, coil 32a) and comprises a relatively resilient stop (320).

FIG.1

FIG.2

FIG.3

FIG.4

FIG. 5

FIG. 6

FIG. 7

FIG.9

FIG. 8

FIG. 10

FIG. 11

FIG. 12

FIG. 13

FIG. 14